Europäisches Patentamt

European Patent Office

Office européen des brevets

(11) Publication number: **0 289 021 B1**

(12) **EUROPEAN PATENT SPECIFICATION**

(45) Date of publication of patent specification: **03.06.92** (51) Int. Cl.⁵: **A61M 29/00, A61B 1/04**

(21) Application number: **88106838.1**

(22) Date of filing: **28.04.88**

(54) **Imaging balloon dilitation catheter.**

(30) Priority: **30.04.87 US 45076**

(43) Date of publication of application:
**02.11.88 Bulletin 88/44**

(45) Publication of the grant of the patent:
**03.06.92 Bulletin 92/23**

(84) Designated Contracting States:
**CH DE FR GB IT LI NL**

(56) References cited:
**EP-A- 0 112 148**
**EP-A- 0 122 089**
**EP-A- 0 177 124**
**WO-A-83/03188**
**US-A- 4 648 892**

(73) Proprietor: **ADVANCED CARDIOVASCULAR SYSTEMS, INC.**
**1395 Charleston Road**
**Mountain View, CA 94039-7101(US)**

(72) Inventor: **Samson, Gene L.**
**32916 Great Salt Lake Street**
**Fremont, California 94587(US)**
Inventor: **Aita, Michael**
**1043 Payette**
**Sunnyvale, California 94087(US)**

(74) Representative: **Baillie, Iain Cameron et al**
**c/o Ladas & Parry, Altheimer Eck 2**
**W-8000 München 2(DE)**

Rank Xerox (UK) Business Services

## Description

### Technical Field

This invention relates to imaging balloon dilatation catheters and more particularly to imaging balloon dilatation catheters for use in angioplasty according to the preamble of claim 1.

### Background

Angioscopes have heretofore been provided by numerous manufacturers for many years, see for example EP-A-0 122 089 and US-A-4 648 892. However, they have not been made small enough so that they can be used with dilatation catheters particularly those which travel over a guidewire. There is therefore a need for a balloon dilatation catheter which has imaging capabilities.

### Brief Description

Briefly and in general terms, the imaging balloon dilatation catheter of the present invention as disclosed in claim 1 is comprised of elongate flexible tubular member having proximal and distal extremities. An inflatable balloon is carried by the tubular member near the distal extremity thereof. The tubular member has a plurality of lumens therein. An illuminating set of optical fibers is disposed in a first of the lumens and extends through the length of the tubular member. An imaging bundle of optical fibers extends through a second of the lumens of the tubular member.

Further, the catheter comprises a third lumen, extending through the dilatation balloon, which has an internal diameter sufficiently large to receive said guide wire and to direct fluid therethrough when said guidewire is disposed within said lumen.

### Drawings

Figure 1 is a schematic block diagram of an imaging balloon dilatation catheter incorporating the present invention and the associated apparatus utilized therewith.

Figure 2 is an enlarged side elevational view of the distal extremity of the imaging balloon dilatation catheter shown in Figure 1 taken along the line 2-2 of Figure 4 and showing the guide wire removed.

Figure 3 is a cross-sectional view taken along the line 3-3 of Figure 2.

Figure 4 is a cross sectional view taken along the line 4-4 of Figure 2.

Figure 5 is a cross sectional view taken along the line 5-5 of Figure 4.

Figure 6 is a cross sectional view similar to Figure 5 showing an alternative embodiment of an imaging balloon catheter incorporating the present invention.

Figure 7 is a cross sectional view taken along the line 7-7 of Figure 6.

### Detailed Description

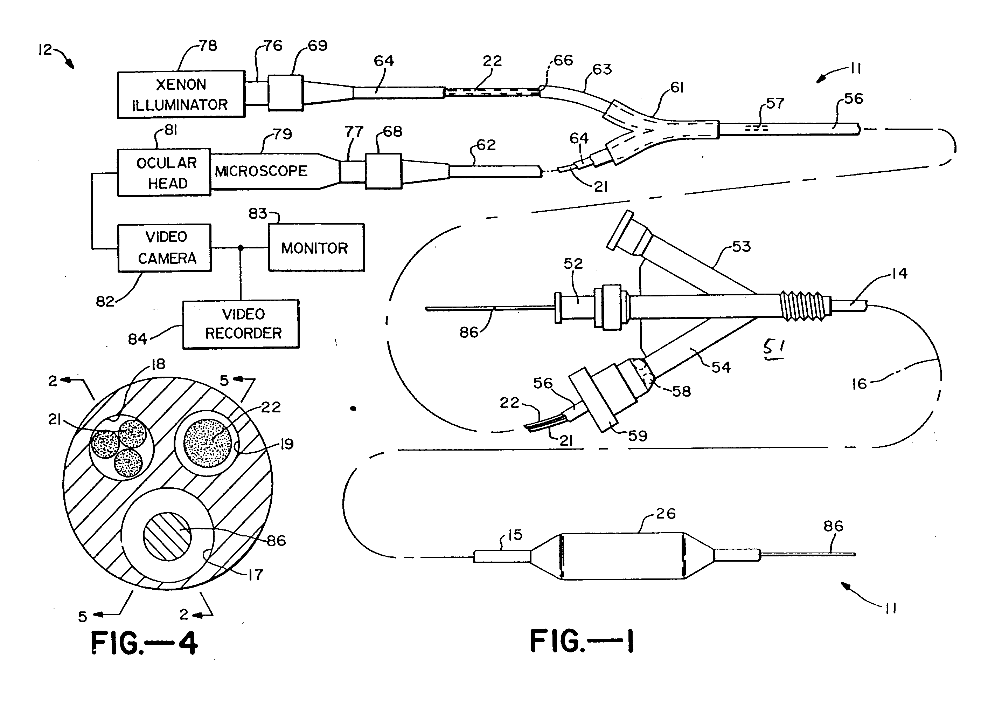

As shown in the drawings, the imaging balloon dilatation catheter and associated apparatus, as shown in Figure 1, is comprised of imaging balloon dilatation catheter 11 and associated apparatus 12.

The imaging balloon dilatation catheter 11 consists of an elongate flexible tubular member 13 which is formed of a suitable material such as polyethylene. The elongate flexible tubular member 13 is provided with proximal and distal extremities 14 and 15 respectively and forms a shaft 16. At least three lumens 17, 18 and 19 are provided in the elongate flexible tubular member 13 with the lumen 17 being identified as a guidewire lumen, the lumen 18 being identified as the illumination optical fiber lumen and the lumen 19 being identified as the imaging optical fiber lumen. The lumens 18 and 19 for reasons hereinafter described can also be identified as inflation/deflation lumens. The guidewire lumen 17 extends throughout the length of the elongate flexible member from the proximal extremity 14 to the distal extremity 15. The lumens 17, 18 and 19 can have suitable diameters as for example the guidewire lumen can have a diameter of .53 mm (.021 inches) and the lumens 18 and 19 a diameter of .38 mm (.015 inches) and the elongate flexible tubular member 13 can have a suitable outside diameter as for example 1.4 mm (.056 inches), alternatively the lumens can all be of the same size. The elongate flexible tubular member 13 can have a suitable length as for example approximately 175 cm to provide the shaft 16 extending between the proximal and distal extremities 14 and 15.

A plurality of discrete illumination optical fibers 21 are provided in the lumen 18 as for example three of such fibers as shown. The illumination optical fibers can be of any suitable type and can be relatively inexpensive. It is only necessary that they have a good bending radius as for example be capable of withstanding bends as small as 1 cm in diameter. They also should have a large numerical aperture as, for example, 0.60.

A bundle 22 of imaging optical fibers is provided in the lumen 19. In order to achieve a suitable image, it is desirable that at least approximately 2,000 optical elements or optical fibers comprise the bundle. The bundle 22 also must have a good bending radius so that the catheter can be utilized for negotiating tortuous vessels in the human body. The type of fibers utilized in the imaging bundle 22 also can be a conventional type as for example of the type disclosed in United States Letters Patent No. 4,135,901.

A gradient index lens 23 is provided at the distal extremity 15 of the elongate flexible tubular member 13. The lens 23 serves to provide an image on its backside which is the same diameter as the bundle 22 which serves to place in that image the entire field of view which is viewed by the lens 23 at the distal extremity of the elongate flexible tubular member 13. The lens 23 is secured to the distal extremity of the bundle 22 by a suitable means such as an epoxy 24. The lens 23 can be recessed by a slight amount as for example 1 mm from the distal extremity of the flexible elongate tubular member 13 so as to protect the lens and also to prevent it from being broken off or scraped off during use of the catheter.

An inflatable balloon 26 is provided on the distal extremity 15 of the elongate flexible tubular member 13. The balloon 26 itself is provided with necked down proximal and distal extremities 27 and 28. The proximal extremity 27 is secured to a necked down portion 13a of the elongate flexible tubular member 13 by suitable means such as an adhesive 29. The necked down portion 13a can be necked down to a suitable diameter as for example 1.19 mm (.047 inches) in outside diameter. The distal extremity 28 of the inflatable balloon 26 can be shrunk onto the distal extremity 15 of the tubular member 13 by application of heat to the same to provide a liquid-type seal. Self-venting means of the type described in United States Patent No. 4,638,805 can be provided for venting the interior 30 of the balloon 26 to ambient. It should be appreciated that if desired instead of a separate balloon an integral balloon can be provided which is formed integral with the elongate flexible tubular member 13.

Means is provided for inflating and deflating the balloon 26 and consists of flow passages 31 and 32 which are provided in the lumens 18 and 19. The flow passages 31 and 32 are provided by the remaining space within the lumens 21 and 22 which are not occupied by the optical fibers 21 in the lumen 18 and the bundle 22 in the lumen 19. Cutouts 33 and 34 are provided in the elongate flexible tubular member 13 within the interior of the balloon 36 which are in communication with the flow passages 31 and 32 respectively. As hereinafter described, the flow passages 31 and 32 can be used for inflating and deflating the balloon 26. Because the cutouts 33 and 34 have been provided, the elongate tubular member 13 within the balloon 26 is of a reduced diameter as for example 1.09 mm (.043 inches).

A pair of balloon markers 38 and 39 are provided near the distal and proximal extremities of the balloon 26. These spaced apart balloon markers 38 and 39 can be formed of a suitable material such as platinum wire wrapped around the bundle 22 of the imaging of optical fibers. An additional marker 41 has been provided for indicating the distal extremity of the catheter 11 and consists of a metallic band for suitable radioopaque material such as gold. The marker 41 is provided over the distal extremity of the tubular member 13 and is encased within the distal extremity 28 of the balloon 26 and is positioned immediately to the rear of the lens 23.

The proximal extremity 14 of the tubular member 13 terminates in a three-arm adapter 51 which has a central arm 52 and side arms 53 and 54. The central arm 53 is in communication with the guidewire lumen 17. Thus the central arm 52 can be utilized for introducing the guidewire or it can be utilized for introducing radiographic contrast agents or flushing agents through the lumen 17. The side arm 53 serves as the balloon inflation/deflation port and is in communication with both of the lumens 18 and 19.

The other side arm 54 carries another elongate flexible tubular member 56 formed of a suitable material such as plastic which can have a suitable diameter as for example an outside diameter of 1.8 mm (.070 inches) and inside diameter of 1 mm (.040 inches). The tubular member 56 extends into the side arm 54 and terminates therein and has the illumination optical fibers 21 and the bundle 22 of imaging fibers extending through the lumen 57 provided therein. An O-ring 58 is provided within the side arm 54 and surrounds the elongate tubular member 56. A knob 59 is mounted on the side arm 54 and is adapted to engage the O-ring 58 to form a liquid tight seal with respect to the elongate tubular member 56 to prevent the escape of blood through the side arm 54.

The elongate tubular member 56 has a suitable length as for example 35 cm. At a junction defined by a Y-shaped reinforcing member 61, the illumination optical fibers 21 and the bundle of imaging fibers 22 are separated and placed in two additional elongate tubular members 62 and 63 having a suitable outside diameter such as 1 mm (.040 inches) and an inside diameter of .76 mm (.030 inches) to provide lumens 64 and 66 extending through the numbers 62 and 63. The illumination optical fibers 21 extend through the lumen 64 in the elongate member 62 and the bundle 22 of imaging optical fibers extends through the lumen 66 of the elongate member 63. The elongate tubular members 62 and 63 have a suitable length as for example 25 cm. The proximal extremities of the flexible elongate members 62 and 63 and the optical fibers 21 and 22 carried therein are connected to conventional type connectors utilized in fiberoptics as for example male-type connectors 68 and 69 identified as SMA. The connectors 68 and 69 are adapted to be mated with female SMA connectors 76 and 77 respectively. The connector 76 is mounted on Xenon illuminator 78 of a conventional type which supplies light to the illumination optical fibers 21. The connector 77 is adapted to be mounted on XYZ stage (not shown) of a microscope 79 which is provided with an ocular head 81 which can be used for viewing by the human eye and also for coupling into a video camera 82. The video camera 82 produces a signal which is supplied to a video monitor 83 and a video recorder 84.

Operation and use of the catheter 11 and the associated apparatus 12 may now be briefly described as follows. The balloon 26 is inflated outside the patient's body by introducing a balloon inflation medium through the balloon inflation port 53. Any air in the balloon is vented through the self-vent holes (not shown). The vent holes are of a size that will permit the excape of air but will not prevent the escape of liquid therethrough. As soon as the air has been removed from the balloon 26 the balloon 26 can be deflated.

Let it be assumed that it is desired to perform an angioplasty with the catheter 11. The catheter 11 can be introduced in the conventional manner over a guidewire 86 which is introduced through the central arm 52 and through the guidewire lumen 17 until it extends beyond the distal extremity of the same. The tip of the guidewire 86 is advanced into the stenosis to be treated and thereafter, the balloon 26 is advanced over the guidewire and is properly positioned within the stenosis. The positioning of the guidewire 86 and the catheter 11 can be observed on a fluoroscope. The balloon 26 is then inflated by introducing a suitable balloon inflation medium through the side arm 53 so that it can pass through the flow passages 31 and 32 provided in the lumens 18 and 19. After the balloon 26 has been inflated to the desired pressure for a suitable period of time, it is again deflated and the balloon 26 is withdrawn from the stenosis with the guidewire still in place. The balloon 26 is then reinflated proximal of the stenosis to block the flow of blook through the stenosis. The guidewire 86 is then removed from the catheter and a suitable flushing medium such as a saline solution is introduced through the guidewire and flushing lumen 17 so that a clear area is produced in front of the lens 23.

Assuming that the apparatus which is shown in Figure 1 is connected to the catheter 11, suitable illumination is provided in the area of the stenosis through the illumination optical fibers 21. The image which is seen by the lens 23 to be viewed through the fiberoptic bundle 22 can be viewed through the monitor 83 or directly through viewing the ocular head 81 by the human eye. At the same time, a video recording can be made on the video recorder 84 of what is being seen. If the opening which has been formed in the stenosis and being imaged and viewed is satisfactory, the balloon 26 can be deflated and the catheter 11 removed from the vessel of the patient. However it is found that the opening of the stenosis is not satisfactory, the guidewire 86 can then again be reinserted into the central arm 52 through the guidewire lumen 17 and through the stenosis. The balloon 26 of the catheter 11 can then be readvanced and the balloon can be reinflated to a suitable pressure for a suitable period of time to see if it is possible to further enlarge the opening in the stenosis. Thereafter, the balloon can be deflated and removed to a region just proximal of the stenosis and reinflated to stop the flow of blood. The guidewire 86 again can be removed and a flushing solution introduced thrugh the lumen 17 to provide a clear field of view for the lens 23. The size of the opening through the stenosis can again be observed to see whether or not the size of the opening has been increased to provide a more satisfactory flow through the stenosis. If necessary, the procedure hearinbefore described can be repeated to ensure that a sufficient enlargement has been obtained.

If desired, a pressure measurement of the blood flow can be made by measuring the pressure at the distal extremity of the catheter 11 by use of the lumen 17. In addition it should be appreciated that the video signal which is supplied from the video camera 82 can be supplied to computer so that it can be computer enhanced if that is desirable.

After the desired procedures have been completed the catheter 11 can be removed from the patient to complete the procedure.

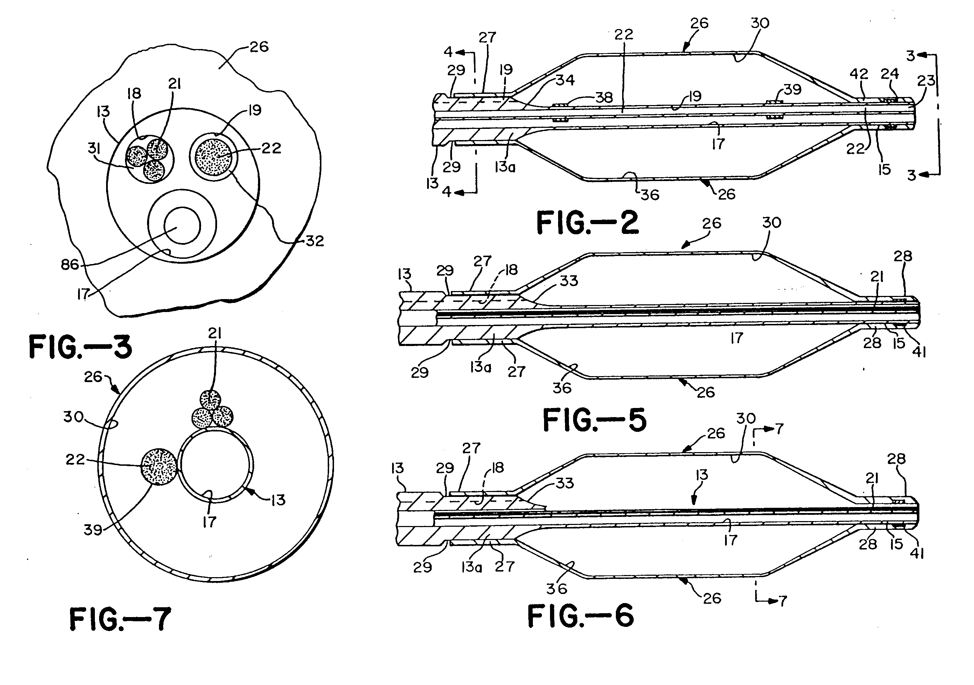

An alternative embodiment of the imaging balloon dilatation catheter incorporating the present invention is shown in Figures 6 and 7. As shown therein, this catheter is very similar to that shown in Figures 2, 3, 4 and 5, with the exception that the plastic which forms the lumens 18 and 19 is removed from the interior of the balloon at the distal extremity 28. This makes it possible to reduce the outside diameter of the distal extremity from the range of 1.52 to 1.57 mm (.060 to .062 inches) for the embodiments shown in Figures 2-5 to the range of 1.32 to 1.37 mm (.052 to .054 inches) for the embodiment which is shown in Figures 6 and 7. In addition, the elimination of this plastic within the balloon decreases the stiffness of the distal extremity of the catheter facilitating negotiation of tortuosities in the vessel in which the catheter is introduced.

In fabricating the construction shown in Figures 6 and 7, rather than removing the plastic which forms the lumens 18 and 19 within the balloon 26, before mounting the balloon on the elongate flexible tubular member 13, the elongate flexible tubular member 13 can be terminated by cutting off the same at a point distal to the interior of the balloon 26. A separate tubular member (not shown) which can serve as a guide wire lumen 17 is provided and can be bonded to the tubular member 13 by suitable means such as an adhesive or by heat staking. Thereafter, the lumination optical fibers 21 can be introduced through the lumen 18 until they extend beyond the point where the lumen terminates and to the distal extremity of the additional tubular member (not shown) which has been secured to the tubular member 13. Similarly, the bundle 22 of imaging optical fibers 21 can be inserted through the imaging optical fiber lumen 19 in a similar manner so that it extends beyond the lumen 19 to the distal extremity of the additional tubular member. Thereafter, the balloon 26 can have its proximal extremities secured to the flexible elongate tubular member 13. The distal extremity 28 of the balloon 26 can then be bonded to the distal extremity of the additional tubular member by suitable means such as an adhesive. As can be seen, this construction will also provide a reduced cross-sectional area at the distal extremity of the catheter as, for example, by reducing the diameter by the same amounts as specified for the embodiments shown in Figures 6 and 7. Such as embodiment will also have reduced stiffness and increased flexibility for negotiating sharp tortuous regions in vessels, as for example, coronary vessels. The use and operation of the device shown in Figures 6 and 7 is identical to that hereinbefore described for the previous embodiment.

It is apparent from the foregoing that the present invention provides an imaging balloon dilatation catheter which makes it possible to perform angioplasty procedures in a conventional manner but which in addition makes it possible immediately thereater to image the results of the procedure to see whether or not it is satisfactory and if not, to permit a further attempt to further enlarge the opening in the stenosis before withdrawing the catheter from the vessel of the patient. The catheter is contstructed in such a manner so that it can negotiate rather tortuous vessels and is particularly adapted for use in the cardiovascuar system although other vessels in the vascular system of a patient, including very small vessels, can be treated in the same manner with the catheter.

## Claims

1. An Imaging balloon dilatation catheter for use with a guidewire (86) comprising:
an elongate flexible tubular member (13) having proximal (14) and distal (15) extremities, said tubular member including a plurality of lumens (17, 18, 19);
an inflatable dilatation balloon (26) carried by said tubular member near the distal extremity thereof;
an illuminating optical fibor moans disposed in a first of the lumens (18) and extending through the length of the tubular member;
an imaging optical fiber means extending through a second of the lumens (19) of the tubular member;
a lens (23) for supplying an image to said imaging optical fibors and carried by the distal extremity of the tubular member;
at least one of said first or second lumens (18, 19) in said tubular means being in communication with the interior of the balloon for inflating and deflating the balloon; characterized in that
said illuminating optical fiber means comprises an illuminating set of optical fibers (21) and in that said imaging optical fiber means comprises an imaging bundle of optical fibers;
further characterized in that the first and second lumens are non-coaxial in relation to each other;
further characterized in that the dilatation balloon (26) is located between the proximal and distal ends of the catheter and is disposed near the distal extremity of the tubular member;
and further characterized by a third lumen (17), extending through the dilatation balloon (26), which has an internal diameter sufficiently large to receive said guidewire (86) and to direct fluid therethrough when said guidewire is disposed within said lumen.

2. The catheter as in Claim 1 characterized by an adapter (51) secured to the proximal extremity of the tubular member, said adapter having a plurality of arms (52, 53, 54), one of said arms (53) including an inflation/deflation ports, at least one of the lumens in the flexible elongate member being in communication with the interior of the balloon and with the inflation/deflation port.

3. The catheter as in claim 2 characterized in that the lumens (18, 19) in which the illuminating set of optical fibers (21) and the set of imaging optical fibers (22) are disposed to provide flow passages extending therethrough which are in communication with the interior of the balloon (26) and which are in communication with tho inflation/deflation port (53).

4.  The catheter as in Claim 2 characterized by an additional elongate flexible member (56) extending through one of the arms (54) of the adapter (51), and wherein the illumination optical fibers (21) and the set of imaging optical fibers (22) extend through the additional elongate flexible member (56).

5.  The catheter as in any one of the preceding claims characterized by a self-venting means carried by the distal extremity of the flexible elongate tubular member and the distal extremity of the balloon which permits the escape of air from the interior of the balloon but restricts the escape of liquid from the interior of the balloon.

6.  The catheter as in any one of the preceding claims characterized by markers (38, 39) identifying certain regions of the balloon.

7.  The catheter as in Claim 1 characterized in that said first and said second lumens (18, 19) are adapted to inflate and deflate said balloon (26).

8.  The catheter as in Claim 1 characterized in that said first lumen (18) is adapted to inflate and deflate said balloon (26).

9.  The catheter as in Claim 1 characterized in that said illuminating set of optical fibers further comprises a plurality of optical fibers.

10. The catheter as in Claim 2 characterized by means (54) in said adapter (51) to connect said imaging optical fiber assembly (22) to external viewing means.

11. The catheter as in Claim 2 or 10 characterized by means (54) in said adapter (51) to connect said illuminating optical fiber assembly (21) to an external source of light.

12. The catheter as in Claim 5 characterized by means in said adapter (51) to introduce a flushing liquid into the third lumen.

13. The catheter as in any one of Claims 1-12 characterized in that said lens (23) is attached to the distal end of said imaging optical fiber assembly (22) such that a gap exists between the proximal face of said lens and the distal end of said optical fibers.

14. The catheter as in any one of Claims 1-12 characterized in that said imaging lens (23) is attached to the distal end of said imaging optical fiber assembly (22) by a transparent adhesive material.

15. The catheter as in any one of Claims 1-11 characterized in that said lens (23) is spaced apart from the distal end of said imaging optical fibers (22).

**Revendications**

1.  Cathéter de dilatation à ballon de formation d'une image, destiné à être utilisé avec un fil de guidage (86) et comprenant :
    un organe tubulaire flexible allongé (10) ayant des extrémités proximale (14) et distale (15), l'organe tubulaire ayant plusieurs lumières (17, 18, 19),
    un ballon gonflable de dilatation (26) supporté par l'organe tubulaire près de son extrémité distale,
    un dispositif à fibres optiques d'éclairement, disposé dans une première des lumières (18) et s'étendant sur la longueur de l'organe tubulaire,
    un dispositif à fibres optiques de formation d'image, disposé dans une seconde des lumières (19) de l'organe tubulaire,
    une lentille (23) destinée à transmettre une image aux fibres optiques de formation d'image et supportée par l'extrémité distale de l'organe tubulaire,
    l'une au moins des première et seconde lumières (18, 19) du dispositif tubulaire étant en communication avec l'intérieur du ballon afin que celui-ci puisse être gonflé et dégonflé,
    caractérisé en ce que
    le dispositif à fibres optiques d'éclairement comporte un ensemble de fibres optiques d'éclairement (21) et en ce que le dispositif à fibres optiques de formation d'image comprend un faisceau de fibres optiques de formation d'image,
    caractérisé en outre en ce que la première et la seconde lumières ne sont pas coaxiales l'une à l'autre,
    caractérisé en ce que le ballon de dilatation (26) est disposé entre les extrémités proximale et distale du cathéter et est disposé à proximité de l'extrémité distale de l'organe tubulaire, et
    caractérisé en outre par une troisième lumière (17) disposée dans le ballon de dilatation (26), et ayant un diamètre interne suffisamment grand pour loger le fil de guidage (86) et pour diriger un fluide lorsque le fil de guidage est placé dans la lumière.

2.  Cathéter selon la revendication 1, caractérisé par un adaptateur (51) fixé à l'extrémité proximale de l'organe tubulaire, l'adaptateur ayant plusieurs branches (52, 53, 54), l'une des branches (53) ayant une lumière de gonflage-dégonflage, l'une au moins des lumières de l'organe flexible allongé étant en communication avec l'intérieur du ballon et avec l'orifice de gonflage-dégonflage.

3. Cathéter selon la revendication 2, caractérisé en ce que les lumières (18, 19) dans lesquelles sont disposées le jeu de fibres optiques d'éclairement (21) et le jeu de fibres optiques de formation d'image (22) forment des passages de circulation qui sont en communication avec l'intérieur du ballon (26) et qui sont en communication avec l'orifice de gonflage-dégonflage (53).

4. Cathéter selon la revendication 2, caractérisé par un organe flexible allongé supplémentaire (56) disposé dans l'une des branches (54) de l'adaptateur (51), et dans lequel les fibres optiques d'éclairement (21) et le jeu de fibres optiques de formation d'image (22) sont disposés dans l'organe flexible allongé supplémentaire (56).

5. Cathéter selon l'une quelconque des revendications précédentes, caractérisé par un dispositif de ventilation automatique supporté par l'extrémité distale de l'organe tubulaire allongé flexible et l'extrémité distale du ballon et qui permet à l'air de s'échapper de l'intérieur du ballon, mais empêche le liquide de s'échapper de l'intérieur du ballon.

6. Cathéter selon l'une quelconque des revendications précédentes, caractérisé par des marqueurs (38, 39) identifiant certaines régions du ballon.

7. Cathéter selon la revendication 1, caractérisé en ce que la première et la seconde lumière (18, 19) sont destinées à gonfler et dégonfler le ballon (26).

8. Cathéter selon la revendication 1, caractérisé en ce que la première lumière (18) est destinée à gonfler et dégonfler le ballon (26).

9. Cathéter selon la revendication 1, caractérisé en ce que le jeu de fibres optiques d'éclairement comporte en outre plusieurs fibres optiques.

10. Cathéter selon la revendication 2, caractérisé par un dispositif (54) placé dans l'adaptateur (51) et destiné à raccorder l'ensemble (22) à fibres optiques de formation d'image à un dispositif externe d'observation.

11. Cathéter selon la revendication 2 ou 10, caractérisé par un dispositif (54) placé dans l'adaptateur (51) et destiné à connecter l'ensemble à fibres optiques d'éclairement (21) à une source de lumière extérieure.

12. Cathéter selon la revendication 5, caractérisé par un dispositif placé dans l'adaptateur (51) et destiné à introduire un liquide de lavage dans la troisième lumière.

13. Cathéter selon l'une quelconque des revendications 1 à 12, caractérisé en ce que la lentille (23) est fixée à l'extrémité distale de l'ensemble (22) à fibres optiques de formation d'image afin qu'un espace existe entre la face proximale de la lentille et l'extrémité distale des fibres optiques.

14. Cathéter selon l'une quelconque des revendications 1 à 12, caractérisé en ce que la lentille (23) de formation d'image est fixée à l'extrémité distale de l'ensemble (22) à fibres optiques de formation d'image par une matière adhésive transparente.

15. Cathéter selon l'une quelconque des revendications 1 à 11, caractérisé en ce que la lentille (23) est placée à distance de l'extrémité distale des fibres optiques (22) de formation d'image.

**Patentansprüche**

1. Bilderzeugender Ballondilatationskatheter zur Verwendung mit einem Führungsdraht (86), umfassend: ein längliches, biegsames rohrförmiges Element (13) mit proximalen (14) und distalen (15) Enden, wobei das rohrförmige Element eine Vielzahl von Lichtungen (17, 18, 19) aufweist; einen aufblasbaren Dilatationsballon (26), der auf dem rohrförmigen Element in der Nähe des distalen Endes angebracht ist; eine faseroptische Beleuchtungseinrichtung, die in einer ersten Lichtung (18) angeordnet ist und sich in Längsrichtung durch das rohrförmige Element erstreckt; eine faseroptische Abbildungseinrichtung, die sich durch eine zweite Lichtung (19) des rohrförmigen Elementes erstreckt; eine Linse (23), die ein Bild zu der faseroptischen Abbildungseinrichtung schickt und auf dem distalen Ende des rohrförmigen Elementes angeordnet ist; wobei mindestens eine der ersten oder zweiten Lichtungen (18, 19) in dem rohrförmigen Element mit dem Inneren des Ballons in Verbindung steht, um den Ballon aufzublasen und zu entleeren; dadurch gekennzeichnet, daß die faseroptische Beleuchtungseinrichtung einen Satz optischer Beleuchtungsfasern (21) umfaßt, und daß die faseroptische Abbildungseinrichtung ein Bündel von optischen Abbildungsfasern umfaßt; und ferner gekennzeichnet durch eine dritte Lichtung (27), die sich durch den Dilatationsballon (26) erstreckt, der einen Innendurchmesser aufweist, der groß genug ist, um den Führungsdraht (86) aufzunehmen, und um Flüssigkeit hindurchzuleiten, wenn der Führungsdraht in der Lichtung angeordnet ist.

2. Katheter nach Anspruch 1, gekennzeichnet durch einen Adapter (51), der am proximalen Ende des rohrförmigen Elementes angebracht ist, wobei der Adapter eine Vielzahl von Armen (52, 53, 54) aufweist, von denen ein Arm (53) einen Anschluß zum Aufblasen/Entleeren aufweist, und mindestens eine der Lichtungen in dem biegsamen länglichen Element steht in Verbindung mit dem Inneren des Ballons und mit dem Anschluß zum Aufblasen/Entleeren.

3. Katheter nach Anspruch 2, dadurch gekennzeichnet, daß die Lichtungen (18, 19), in denen die optischen Beleuchtungsfasern (21) und die optischen Abbildungsfasern (22) angeordnet sind, durch sie hindurch verlaufende Fließwege bilden, die mit dem Inneren des Ballons (26) und mit dem Anschluß (53) zum Aufblasen/Entleeren in Verbindung stehen.

4. Katheter nach Anspruch 2, dadurch gekennzeichnet, daß sich ein zusätzliches längliches, biegsames Element (56) durch einen der Arme (54) des Adapters (51) hindurch erstreckt, und daß die optischen Beleuchtungsfasern (21) und die optischen Abbildungsfasern (22) sich durch das zusätzliche längliche, biegsame Element (56) hindurch erstrecken.

5. Katheter nach einem der vorhergehenden Ansprüche, gekennzeichnet durch eine Selbstentlüftungseinrichtung, die auf dem distalen Ende des biegsamen länglichen, rohrförmigen Elementes und auf dem distalen Ende des Ballons angeordnet ist und das Entweichen von Luft aus dem Inneren des Ballons erlaubt, aber das Entweichen von Flüssigkeit aus dem Inneren des Ballons einschränkt.

6. Katheter nach einem der vorhergehenden Ansprüche, gekennzeichnet durch Markierungen (38, 39), die bestimmte Bereiche des Ballons kennzeichnen.

7. Katheter nach Anspruch 1, dadurch gekennzeichnet, daß die ersten und zweiten Lichtungen (18, 19) geeignet sind, den Ballon (26) aufzublasen und zu entleeren.

8. Katheter nach Anspruch 1, dadurch gekennzeichnet, daß die erste Lichtung (18) geeignet ist, den Ballon (26) aufzublasen und zu entleeren.

9. Katheter nach Anspruch 1, dadurch gekennzeichnet, daß die faseroptische Beleuchtungseinheit ferner eine Vielzahl optischer Fasern enthält.

10. Kathether nach Anspruch 2, gekennzeichnet durch eine Einrichtung (54) in dem Adapter (51), welche die faseroptische Abbildungseinheit (22) mit einer außen angeordneten Betrachtungseinrichtung verbindet.

11. Katheter nach Anspruch 2 oder 10, gekennzeichnet durch eine Einrichtung (54) in dem Adapter, welche die faseroptische Beleuchtungseinheit (21) mit einer außen angeordneten Lichtquelle verbindet.

12. Katheter nach Anspruch 5, gekennzeichnet durch eine Einrichtung in dem Adapter (51), welche eine Spülflüssigkeit in die dritte Lichtung einleitet.

13. Katheter nach einem der Ansprüche 1 bis 12, dadurch gekennzeichnet, daß die Linse (23) am distalen Ende der faseroptischen Abbildungseinheit (22) angebracht ist, so daß zwischen der proximalen Seite der Linse und dem distalen Ende der optischen Fasern eine Lücke besteht.

14. Katheter nach einem der Ansprüche 1 bis 12, dadurch gekennzeichnet, daß die Abbildungslinse (23) am distalen Ende der faseroptischen Abbildungseinheit (22) mit Hilfe eines durchsichtigen Klebstoffes befestigt ist.

15. Katheter nach einem der Ansprüche 1 bis 11, dadurch gekennzeichnet, daß die Linse (23) im Abstand von dem distalen Ende der optischen Abbildungsfasern (22) angeordnet ist.

XENON ILLUMINATOR

MICROSCOPE

OCULAR HEAD

MONITOR

VIDEO CAMERA

VIDEO RECORDER

FIG.—1

FIG.—4

FIG.—2

FIG.—5

FIG.—6

FIG.—3

FIG.—7